# EUROPEAN PATENT SPECIFICATION

(11) **EP 3 885 445 B1**
(45) Date of publication and mention of the grant of the patent: **23.08.2023**
(21) Application number: 21157081.7
(22) Date of filing: 13.04.2018
(51) Int. Cl.: C12Q 1/68, C12Q 1/6855

(54) **METHODS OF ATTACHING ADAPTERS TO SAMPLE NUCLEIC ACIDS**
VERFAHREN ZUR BEFESTIGUNG VON ADAPTERN AN PROBENNUKLEINSÄUREN
PROCÉDÉS DE FIXATION D'ADAPTATEURS À DES ACIDES NUCLÉIQUES ÉCHANTILLONS

(30) Priority: 14.04.2017 US 201762485769 P; 14.04.2017 WO PCT/US2017/027809; 18.04.2017 US 201762486663 P; 08.06.2017 US 201762517145 P
(43) Date of publication of application: 29.09.2021
(62) Divisional of application: 18783709.1
(73) Proprietor: Guardant Health, Inc., Redwood City, CA 94063 (US)
(72) Inventor: KENNEDY, Andrew, La Jolla, California 92037 (US); MORTIMER, Stefanie Ann Ward, Morgan Hill, California 95037 (US)
(74) Representative: Carpmaels & Ransford LLP

(56) References cited:
- WO-A1-2015/044262
- WO-A1-2016/135300
- Cheng-Cang Wu ET AL: "Long-span, mate-pair scaffolding and other methods for faster next-generation sequencing library creation", Nature Methods, 1 September 2012 (2012-09-01), pages i-ii, XP055201936, Retrieved from the Internet: URL:http://www.nature.com/nmeth/journal/v9 /n9/pdf/nmeth.f.358.pdf [retrieved on 2015-07-13]

## Description

### BACKGROUND

Cancer is a major cause of disease worldwide. Each year, tens of millions of people are diagnosed with cancer around the world, and more than half eventually die from it. In many countries, cancer ranks the second most common cause of death following cardiovascular diseases. Early detection is associated with improved outcomes for many cancers.

Cancers are often detected by biopsies of tumors followed by analysis of cells, markers or DNA extracted from cells. But more recently it has been proposed that cancers can also be detected from cell-free nucleic acids in body fluids, such as blood or urine (see, e.g., Siravegna et al., Nature Reviews 2017). Such tests have the advantage that they are non-invasive and can be performed without identifying a suspected cancer cells to biopsy. However, the amount of nucleic acids in body fluids is very low. Thus, such analyses require efficient methods to convert native cell-free DNA in body fluids to forms amenable to analysis.

Preparing DNA molecules from patient samples for analysis commonly involves first repairing single-stranded overhangs to permit ligation to adapters for amplification and sequencing. Repair can be effected by digesting the overhanging strand or extending the opposing strand to produce a blunt end followed by phosphorylation of 5' ends and blunt end ligation to adapters. Alternatively, after blunt ending, blunt ends can be A-tailed with a Taq polymerase. A-tailed fragments are annealed and ligated with adapters including a single nucleotide T-tail at a 3' end. This configuration favors the desired adapter-DNA molecule ligation but the overall conversion efficiency of DNA molecules in a sample to molecules that can be sequenced can still be unacceptably low for samples in which only small amounts of nucleic acids are available.

WO 2015/044262 describes enzyme compositions for DNA-end repair, adenylation and phosphorylation. WO 2016/135300 discloses methods and kits to improve the efficiency of next generation sequencing library construction. Wu et. al. (Nat Methods 9, i-ii, 2012) describes methods for faster next-generation sequencing library creation.

### SUMMARY

The invention provides a kit comprising at least partially double-stranded adapters with a single nucleotide T overhang at a 3' end (T-tailed adapters); and at least partially double-stranded adapters with a single nucleotide C overhang at a 3' end (C-tailed adapters).

In some embodiments, the molar ratio of at least partially double-stranded adapters with a single nucleotide T overhang to a single nucleotide C overhang is 4:1 to 2:1, for example 3:1. In some embodiments, the percentage of C-tailed adapters relative to T-tailed adapters ranges from about 5-40% by moles, for example, 10-35%, 15-25%, 20-35%, 25-35% or about 30%.

In some embodiments, the kit further comprises at least partially double-stranded blunt-ended adapters. In some embodiments, the molar ratio of blunt-ended adapters to tailed adapters is 1:5 to 1:500, for example 1:10 to 1:100. In some embodiments, blunt-ended adapters are at a molar ratio of 0.2-20%, or 0.5-15%, or 1-10% of adapters with T- and C-tailed adapters.

In some embodiments, the kit further comprises a T4 polymerase or Klenow large fragment; and/or a Taq polymerase. In some embodiments, the kit further comprises four standard nucleotide types.

In some embodiments, the kit is free of at least partially double-stranded adapters with A single nucleotide 3' tails, and at least partially double-stranded adapters with G single nucleotide 3' tails.

In some embodiments, the adapters are Y-shaped adapters. In some embodiments, the adapters comprise oligonucleotides of SEQ ID NOs 1 and 2, and 3 and 2.

In some embodiments, the adapters comprise a primer binding site and/or a barcode. In some embodiments, the barcodes provide between 64 and 90,000, between 400 and 22,500, between 400 and 14,400, or between 900 and 14,400 different barcode combinations.

A kit of the invention may be useful in a method of preparing nucleic acids for analysis comprising;
(a) blunt-ending double-stranded nucleic acids with single- stranded overhangs in a sample by the action of one or more enzymes providing a 5'-3' polymerase activity and 3'-5' proof reading activity, and four standard nucleotide types, wherein single-stranded overhangs with 5' ends serve as templates for extension of a complementary strand by the polymerase activity and single-stranded overhangs with 3' ends are digested by the proof reading activity producing blunt-ended nucleic acids; (b) without separating the blunt-ended nucleic acids from other components of the sample, end-tailing the blunt-ended nucleic acids by action of a polymerase without a 3'-5' proof reading function, which performs a non-template directed addition of a nucleotide to the 3' ends of blunt-ended nucleic acids, wherein A is added preferentially to G preferentially to C or T; (c) annealing the nucleic acids from step (c) with at least partially double-stranded adapters with a single nucleotide T or C overhangs at a 3'-end; and (d) ligating the nucleic acids to the adapters. Optionally, the method further comprises denaturing the one or more enzymes after step (a). Optionally, the method further comprises contacting the sample with the one or more enzymes, the four standard nucleotide types and the polymerase without a 3'-5' proof reading function. Optionally, the sample is contacted with the one or more enzymes, the four standard nucleotide types and the polymerase without a 3'-5' proof reading function together. Optionally step (b) is performed at a higher temperature than step (a). Optionally, step (a) is performed at ambient temperature and step (b) at a temperature over 60 C. Optionally, the one or more enzyme are a polymerase with 5'-3' polymerase activity and 3'-5' proof reading activity. Optionally the polymerase without a 3'-5' proof reading function is a thermostabile polymerase and the method further comprises increasing temperature of the sample after step (a) to inactivate the polymerase with 5'-3' polymerase activity and 3'-5' proof reading activity. Optionally the method further comprises (e) amplifying the nucleic acids ligated to the adapters; and (f) analyzing the nucleic acids.

Optionally the method further comprises contacting the sample with at least partially double-stranded blunt-ended adapters, which ligate with blunt-ended double-stranded nucleic acids which have not undergone the non-template directed addition of a nucleotide to the 3' ends in the ligating step. Optionally, the first polymerase is T4 polymerase or Klenow large fragment. Optionally, the second polymerase is a Taq polymerase. Optionally at least steps (a)-(e) are performed in a single tube. Optionally, wherein steps (a)-(f) or (a) to (g) are performed in a single tube. Optionally, the molar ratio of at least partially double-stranded adapters with a single nucleotide T to a single nucleotide C is 4:1 to 2:1, preferably 3:1. Optionally, the molar ratio of blunt ended adapters to tailed adapters is 1:5 to 1:500, preferably 1: 10 to 1:100. Optionally, at least 70% of the double-stranded nucleic acids in the sample are joined to adaptors. Optionally, at least 70% of the available double-stranded nucleic acids in the sample are analyzed. Optionally, step (f) comprises sequencing the nucleic acids ligated to the adapters. Optionally, the sequencing sequences a nucleotide that formed an overhang in step (c) or (d).

A kit of the invention may further be useful in a method of converting double-stranded DNA into adapter-tagged DNA comprising: (a) contacting a population of double-stranded DNA molecules with a population of at least partially double-stranded adapters, wherein: (i) the population of double-stranded DNA molecules comprises DNA molecules comprising a single nucleotide A overhang and DNA molecules comprising a single nucleotide G overhang, and wherein single nucleotide A overhangs are more abundant (e.g., 10 times, 100 times, 1000 times) than single nucleotide G overhangs in the population, and (ii) the population of at least partially double-stranded adapters comprises adapters comprising a single nucleotide T overhang and adapters comprising a single nucleotide C overhang; and (b) ligating the adapters to the DNA molecules, wherein ligating produces adapter-tagged DNA.

Optionally, (i) the population of double-stranded DNA molecules further comprises at least one of: DNA molecules comprising a single nucleotide C overhang, DNA molecules comprising a single nucleotide T overhang and a blunt end, and (ii) the population of at least partially double-stranded adapters further comprises at least one of: adapters comprising a single nucleotide G overhang, adapters comprising a single nucleotide A overhang and a blunt end. Optionally, the at least partially double-stranded adapters comprise an NGS ("next-generation sequencing") primer binding site and a DNA barcode. Optionally, the population of the at least partially double-stranded adapters comprise a plurality of different DNA barcodes. Optionally, the number of barcode combinations attachable to both ends of a double-stranded DNA molecule is less than the number of double-stranded DNA molecules in the population, e.g., between 5 and 10,000 different combinations. Optionally, the method, further comprises: (c) amplifying the adapter tagged DNA using amplification primers comprising a sample index barcode and a nucleotide sequence adapted to hybridize to an oligonucleotide immobilized to a flow cell support. Optionally, the adapters are Y-shaped adapters. Optionally, the sample is a bodily fluid sample, such as whole blood, serum, or plasma. Optionally, the nucleic acid population is a cell-free nucleic acid population. Optionally, the sample is from a subject suspected of having a cancer. Optionally, the analyzing step detects a somatic or germline variant, a copy number variation, a single nucleotide variation (SNV), and indel or gene fusion.

A population of adapted nucleic acids may be produced using a kit of the invention, the population comprising a plurality of nucleic acid molecules each of which comprises a nucleic acid fragment flanked on both sides by an adapter including a bar code with an A/T or G/C base pair between the nucleic acid fragment and adapter. The plurality of nucleic acid molecules may be at least 100,000 molecules. The ratio of A/T base pairs to G/C base pairs may be between 2:1 and 4:1. At least 99 % of nucleic acid molecules in the population may have a nucleic acid fragment flanked by adapters with different bar codes.

### BRIEF DESCRIPTION OF THE FIGURE

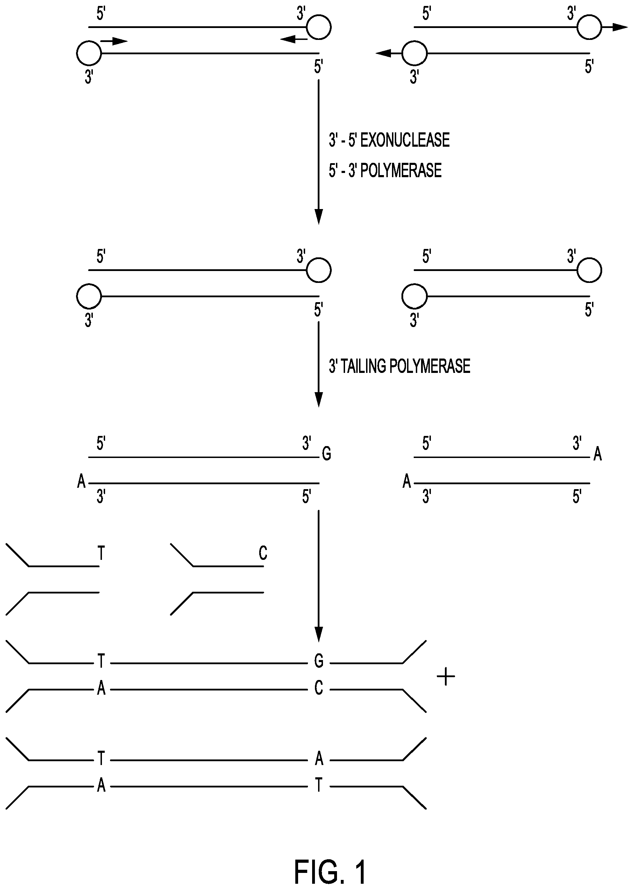

Fig. 1 shows blunt-ending, end-tailing and joining to -T and -C tailed Y-shaped adapters of sample DNA.

### DEFINITIONS

A subject refers to an animal, such as a mammalian species (preferably human) or avian (e.g., bird) species, or other organism, such as a plant. More specifically, a subject can be a vertebrate, e.g., a mammal such as a mouse, a primate, a simian or a human. Animals include farm animals, sport animals, and pets. A subject can be a healthy individual, an individual that has or is suspected of having a disease or a predisposition to the disease, or an individual that is in need of therapy or suspected of needing therapy.

A genetic variant refers to an alteration, variant or polymorphism in a nucleic acid sample or genome of a subject. Such alteration, variant or polymorphism can be with respect to a reference genome, which may be a reference genome of the subject or other individual. Variations include one or more single nucleotide variations (SNVs), insertions, deletions, repeats, small insertions, small deletions, small repeats, structural variant junctions, variable length tandem repeats, and/or flanking sequences, copy number variants (CNVs), transversions and other rearrangements are also forms of genetic variation. A variation can be a base change, insertion, deletion, repeat, copy number variation, transversion, or a combination thereof.

A cancer marker is a genetic variant associated with presence or risk of developing a cancer. A cancer marker can provide an indication a subject has cancer or a higher risk of developing cancer than an age and gender matched subject of the same species. A cancer marker may or may not be causative of cancer.

A nucleic acid tag is a short nucleic acid (e.g., less than 100, 50 or 10 nucleotides long), usually of artificial sequence and usually DNA, used to label sample nucleic acids to distinguish nucleic acids from different samples (e.g., representing a sample index), of different types, or which have undergone different processing. Tags can be single- or double-stranded. Nucleic tags can be decoded to reveal information such as the sample of origin, form or processing of a nucleic acid. Tags can be used to allow pooling and parallel processing of multiple nucleic acids bearing different tags with the nucleic acids subsequently being deconvoluted by reading the tags. Tags can also be referred to as molecular identifiers or barcodes.

Adapters are short nucleic acids (e.g., less than 500, 100 or 50 nucleotides long and typically DNA) usually at least partly double-stranded for linkage to either or both ends of a sample nucleic acid molecule. Adapters can include primer binding sites to permit amplification of a sample nucleic acid molecule flanked by adapters at both ends, and/or a sequencing primer binding site, including primer binding sites for next generation sequences. Adapters can also include binding sites for capture probes, such as an oligonucleotide attached to a flow cell support. Adapters can also include a tag as described above. Tags are preferably position relative to primer and sequencing primer binding sites, such that a tag is included in amplicons and sequencing reads of a sample nucleic acid. The same or different adapters can be linked to the respective ends of a sample molecule. Sometimes the same adapter is linked to the respective ends except that the tag is different. A preferred adapter is a Y-shaped adapter in which one end is blunt ended or tailed as described herein, for joining to a sample nucleic acid, which is also blunt ended or tailed with a complementary nucleotide. Another preferred adapter is a bell-shaped adapter, likewise with a blunt or tailed end for joining to a nucleic acid to be analyzed.

The four standard nucleotide types refer to A, C, G, T for deoxyribonucleotides and A, C, T and U for ribonucleotides.

### DETAILED DESCRIPTION

### 1. General

Sample preparation for new generation sequencing platforms often follows a similar protocol. Samples typically contain double-stranded nucleic acid fragments with single-stranded overhangs. Such fragments can be blunt-ended and ligated to adapters directly. But such ligations also result in byproducts in which adapters or fragments form concatemers. Formation of such byproducts can be reduced by an alternative procedure in which blunt-ended fragments are A-tailed and ligated to T-tailed adapters. Commercial kits that perform end repair and tailing in a single tube are simple to use and fast and can be used with commercially available adaptors. (For example, NEBNext Ultra II (New England Biolabs, Ipswich, MA.). However, use of kits not optimized for A-tailing can result in tailing with other nucleotides, such as G, T and C. The result of inefficient tailing is inefficient ligation of adapters and low complexity libraries.

A kit of the invention may be useful in improved methods of preparing double-stranded nucleic acids (preferably DNA) with single-stranded overhangs for amplification and subsequent analysis, particularly sequencing. It has been found that contacting blunt-ended double-stranded nucleic acids with Taq in the presence of all four standard nucleotide types results in non-templated directed addition of a single nucleotide to the 3' ends of the nucleic acid such that A is added most frequently followed by G followed by C and T. Although inclusion of additional nucleic acid molecules increases the potential for off-target side reactions, it has been found that the proportion of single-G tailing is sufficiently high relative to single-A tailing that the efficiency of ligation of nucleic acid molecules in a sample to adapters can be significantly increased by including a customized mix of adapters tailed not only with T (as in prior methods) but also with C, which adapters anneal respectively to 3' ends of DNA molecules tailed with A and G. The ligation efficiency can be increased even further by also including blunted-ended adapters (i.e., not tailed with any nucleotide) to ligate to blunt-ended nucleic acid molecules in the sample that have failed to undergo tailing with any nucleotide.

### 2. Samples

A sample can be any biological sample isolated from a subject. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids.

The volume of plasma can depend on the desired read depth for sequenced regions. Exemplary volumes are 0.4-40 mL, 5-20 mL, 10-20 mL. For examples, the volume can be 0.5 mL, 1 mL, 5 mL 10 mL, 20 mL, 30 mL, or 40 mL. A volume of sampled plasma may be for example 5 to 20 mL.

A sample can comprise various amount of nucleic acid that contains genome equivalents. For example, a sample of about 30 ng DNA can contain about 10,000 haploid human genome equivalents and, in the case of cell-free DNA, about 200 billion individual nucleic acid molecules. Similarly, a sample of about 100 ng of DNA can contain about 30,000 haploid human genome equivalents and, in the case of cell-free DNA, about 600 billion individual molecules. Some samples contain 1-500, 2-100, 5-150 ng cell-free DNA, e.g., 5-30 ng, or 10-150 ng cell-free DNA.

A sample can comprise nucleic acids from different sources. For example, a sample can comprise germline DNA or somatic DNA. A sample can comprise nucleic acids carrying mutations. For example, a sample can comprise DNA carrying germline mutations and/or somatic mutations. A sample can also comprise DNA carrying cancer-associated mutations (e.g., cancer-associated somatic mutations).

Exemplary amounts of cell-free nucleic acids in a sample before amplification range from about 1 fg to about 1 ug, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng.

An exemplary sample is 5-10 ml of whole blood, plasma or serum, which includes about 30 ng of DNA or about 10,000 haploid genome equivalents.

Cell-free nucleic acids are nucleic acids not contained within or otherwise bound to a cell or in other words nucleic acids remaining in a sample of removing intact cells. Cell-free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. Double-stranded DNA molecules at least some of which have single-stranded overhangs are a preferred form of cell-free DNA for any method disclosed herein. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells.

A cell-free nucleic acid can have one or more epigenetic modifications, for example, a cell-free nucleic acid can be acetylated, methylated, ubiquitinylated, phosphorylated, sumoylated, ribosylated, and/or citrullinated.

Cell-free nucleic acids have a size distribution of about 100-500 nucleotides, particularly 110 to about 230 nucleotides, with a mode of about 168 nucleotides and a second minor peak in a range between 240 to 440 nucleotides.

Cell-free nucleic acids can be isolated from bodily fluids through a partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica based columns to remove contaminants or salts. Non-specific bulk carrier nucleic acids, for example, may be added throughout the reaction to optimize certain aspects of the procedure such as yield.

After such processing, samples can include various forms of nucleic acid including double-stranded DNA, single-stranded DNA and single-stranded RNA. Optionally, single stranded DNA and RNA can be converted to double stranded forms so they are included in subsequent processing and analysis steps.

### 3. Linking sample nucleic acid molecules to adapters

Nucleic acid present in a sample with or without prior processing as described above typically contain a substantial portion of molecules in the form of partially double-stranded molecules with single-stranded overhangs. Such molecules can be converted to blunt-ended double-stranded molecules by treating with one or more enzymes to provide a 5'-3' polymerase and a 3'-5' exonuclease (or proof reading function), in the presence of all four standard nucleotide types as shown in Fig. 1, upper. Such a combination of activities can extend strands with a recessed 3' end so they end flush with the 5' end of the opposing strand (in other words generating a blunt end) or can digest strands with 3' overhangs so they are likewise flush with the 5' end of the opposing strand. Both activities can optionally be conferred by a single polymerase. The polymerase is preferably heat-sensitive so that its activity can be terminated when the temperature is raised. Klenow large fragment and T4 polymerase are examples of suitable polymerase.

The one or more enzymes conferring 5'-3' polymerase and a 3'-5' exonuclease activity are preferably denatured by raising the temperature or otherwise. For example, denaturation can be effected by raising the temperature to e.g., to 75°-80° C. The samples are then acted on by a polymerase lacking a proof reading function (Fig. 1 middle). This polymerase is preferably thermostabile such as to remain active at the elevated temperature. Taq, Bst large fragment and Tth polymerases are examples of such a polymerase. The second polymerase effects a non-templated addition of a single nucleotide to the 3' ends of blunt-ended nucleic acids. Although the reaction mixture typically contains equal molar amounts of each of the four standard nucleotide types from the prior step, the four nucleotide types are not added to the 3' ends in equal proportions. Rather A is added most frequently, followed by G followed by C and T.

After tailing of the sample molecules, and with or without subsequent purification of the tailed sample molecules, the tailed sample molecules are contacted with adapters tailed with complementary T and C nucleotides at one end of the adapters (Fig. 1, lower). Adapters are typically formed by separate synthesis and annealing of their respective strands. The additional T and C tails can thus be added as an extra nucleotide in synthesis of one of the strands. Typically adapters tailed with G and A are not included because although these adapters might anneal with sample molecules tailed with C and T respectively, they would also anneal with other adapters. Adapter molecules and sample molecules bearing complementary nucleotides (i.e., T-A and C-G) at their 3' ends anneal and can be ligated to one another. The percentage of C-trailed adapters relative to T-tailed adapters ranges from about 5-40% by moles, for example, 10-35%, 15-25%, 20-35%, 25-35% or about 30%. Because the non-template directed addition of a single nucleotide to the 3' ends of sample molecules does not proceed to completion, the sample also contains some blunt-ended sample molecules without tailing. These molecules can be recovered by also supplying the sample with adapters having one and preferably only one blunt end. Blunt end adapters are usually supplied at a molar ratio of 0.2-20%, or 0.5-15% or 1-10% of adapters with T- and C-tailed adapters. Blunt-ended adapters can be provided at the same time, before or after the T- and C-tailed adapters. Blunt-ended adapters ligated with blunt-ended sample molecules again resulting in sample molecules flanked on both sides by adapters. These molecules lack the A-T or C-G nucleotide pairs between sample and adapters present when tailed sample molecules are ligated to tailed adapters.

The adapters used in these reactions preferably have one and only one end tailed with T or C or one and only one end blunt so that they can ligate with sample molecules in only one orientation. The adapters can be for example Y-shaped adapters in which one end is tailed or blunt and the other end has two single strands. Exemplary Y-shaped adapters have sequences as follows with (6 bases) indicating a tag. The upper oligonucleotide includes a single base T tail.

Universal Adapter:

5’AATGATACGGCGACCACCGAGATCTACACTCTTTCCCTACACGACGCTCTTCCGAT
CT (SEQ ID NO. 1).

Adapter, Index 1-12: 5' GATCGGAAGAGCACACGTCTGAACTCCAGTCAC (6 bases) ATCTCGTATGCCGTCTTCTGCTTG (SEQ ID NO. 2)

Another Y-shaped adapter with a C tail has the sequences:
5'AATGATACGGCGACCACCGAGATCTACACTCTTTCCCTACACGACGCTCTT CCGATCC (SEQ ID NO. 3) and Adapter, Index 1-12: 5' GATCGGAAGAGCACACGTCTGAACTCCAGTCAC (6 bases) ATCTCGTATGCCGTCTTCTGCTTG (SEQ ID NO. 2)

Customized combinations of such oligonucleotide including oligonucleotides with both T and C tails can be synthesized for use in the present methods.

A truncated version of these adapter sequences has been described by Rohland et al., Genome Res. 2012 May; 22(5): 939-946.

Adapters can also be bell-shaped with only one end, which is tailed or blunt. Adapters can include a primer binding site for amplification, a binding site for a sequencing primer, and/or a nucleic acid tag for purposes of identification. The same or different adapters can be used in in a single reaction.

When adapters include an identification tag and nucleic acids in a sample are attached to adapters at each end, the number of potential combinations of identifiers increases exponentially with the number of unique tags supplied (i.e., nⁿ combinations, where n is the number of unique identification tags). In some methods, the number of combinations of unique tags is sufficient that it is statistically probable that all or substantially all (e.g., at least 90%) of different double-stranded DNA molecules in the sample receive a different combination of tags. In some methods, the number of unique combinations of identifier tags is less than the number of unique double-stranded DNA molecules in the sample (e.g., 5-10,000 different tag combinations).

A kit providing suitable enzymes for performing the above methods is the NEBNext^{®} Ultra^{™} II DNA Library Prep Kit for Illumina^{®}. The kit provides the following reagents

NEBNext Ultra II End Prep Enzyme Mix , NEBNext Ultra II End Prep Reaction Buffer, NEBNext Ligation Enhancer, NEBNext Ultra II Ligation Master Mix -20, NEBNext^{®} Ultra II Q5^{®} Master Mix.

The blunt-ending and tailing of sample nucleic acids can be performed in a single-tube. Blunt-ended nucleic acids need not be separated from the enzyme(s) performing the blunt ending before the tailing reaction occurs. Optionally, all enzymes, nucleotides and other reagents are supplied together before the blunt ending reaction occurs. Supplying together means that all are introduced in the sample sufficiently proximate in time such that all are present when the sample incubation occurs for blunt ending to take place. Optionally, nothing is removed from the samples after supplying the enzymes, nucleotides and other reagents at least until both the blunt ending and end tailing incubations have been completed. Often, the end tailing reaction is performed at a higher temperature than the blunt ending reaction. For example, the blunt ending reaction can be performed at ambient temperature in which the 5'-3' polymerase and 3'-5' exonuclease are active and the thermostabile polymerase is inactive or minimally active, and the end tailing reaction performed at an elevated temperature, such as over 60°C, when the 5'-3' polymerase and 3'-5' exonuclease are inactive and the thermostabile polymerase is active.

Attachment of T- and C-tailed adapters as described results in a population of adapted nucleic acids the population comprising a plurality of nucleic acid molecules each of which comprises a nucleic acid fragment flanked on both sides by an adapter including a bar code with an A/T or G/C base pair between the nucleic acid fragment and adapter. The plurality of nucleic acid molecules can be at least, 10,000, 100,000 or 1,000,000 molecules. The ratio of A/T base pairs to G/C base pairs at junction regions between fragments and flanking adapters depends on the ratio of T- to C-tailed adapters and be for example between 2:1 and 4:1. Most nucleic acids in the population are flanked by adapters with different bar codes (e.g., at least 99 %). If blunt ended adapters are also included, then the population includes nucleic acid molecules in a nucleic acid fragment is directly joined at either or both ends to an adapter (i.e., no intervening A/T or G/C pair).

### 4. Amplification

Sample nucleic acids flanked by adapters can be amplified by PCR and other amplification methods typically primed from primers binding to primer binding sites in adapters flanking a nucleic acid to be amplified. Amplification methods can involve cycles of extension, denaturation and annealing resulting from thermocycling or can be isothermal as in transcription mediated amplification. Other amplification methods include the ligase chain reaction, strand displacement amplification, nucleic acid sequence based amplification, and self-sustained sequence based replication.

Preferably, methods in which kits of the invention may be used result in at least 75, 80, 85, 90 or 95% of double-stranded nucleic acids in the sample being linked to adapters. Preferably use of T- and C-tailing increases the percentage of double-stranded nucleic acids in the sample linked to adaptors relative to control methods performed with T-tailed adapters alone by at least 1, 2, 3, 4, 5, 6, 7, 8, 9 or 10% (an increase of yield from 75% to 80% being considered a 5% increase). Preferably, use of T- and C-tailing in combination with blunt-ended adaptors increase the percentage of double-stranded nucleic acids linked to adaptors by at least 5, 10, 15, 20 or 25%. The percentage of nucleic acids linked to adaptors can be determined by comparative gel electrophoresis of the original sample and the processed sample after linkage to adapters has been completed.

Preferably, methods in which kits of the invention may be used result in at least 75, 80, 85, 90 or 95% of available double-stranded molecules in the sample being sequenced. Preferably the use of T- and C-tailing increases the percentage of double-stranded nucleic acids in the sample being sequenced relative to control methods performed with T-tailed adapters alone by at least 1, 2, 3, 4, 5, 6, 7, 8, 9 or 10%. Preferably the use of T- and C-tailing in combination with blunt ended adaptors increases the percentage of double-stranded nucleic acid in the sample being sequenced relative to control methods performed with T-tailed adaptors along by at least 5, 10, 15, 20 or 25%. The percentage of nucleic acids being sequenced can be determined by comparing the number of molecules actually sequenced based on the number that could have been sequenced based on the input nucleic acids and regions of the genome targeted for sequencing.

### 5. Tags

Tags providing molecular identifiers or bar codes can be incorporated into or otherwise joined to adapters by ligation, overlap extension PCR among other methods. Generally, assignment of unique or non-unique identifiers, or molecular barcodes in reactions follows methods and systems described by US patent applications 20010053519, 20030152490, 20110160078, and U.S. Pat. No. 6,582,908 and U.S. Pat. No. 7,537,898.

Tags can be linked to sample nucleic acids randomly or non-randomly. In some cases, they are introduced at an expected ratio of unique identifiers to microwells. For example, the unique identifiers may be loaded so that more than about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers are loaded per genome sample. In some cases, the unique identifiers may be loaded so that less than about 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers are loaded per genome sample. In some cases, the average number of unique identifiers loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers per genome sample.

In some cases, unique identifiers may be predetermined or random or semi-random sequence oligonucleotides. In other cases, a plurality of barcodes may be used such that barcodes are not necessarily unique to one another in the plurality. In this example, barcodes may be ligated to individual molecules such that the combination of the bar code and the sequence it may be ligated to creates a unique sequence that may be individually tracked. As described herein, detection of non-unique barcodes in combination with sequence data of beginning (start) and end (stop) portions of sequence reads may allow assignment of a unique identity to a particular molecule. The length, or number of base pairs, of an individual sequence read may also be used to assign a unique identity to such a molecule. As described herein, fragments from a single strand of nucleic acid having been assigned a unique identity may thereby permit subsequent identification of fragments from the parent strand.

Polynucleotides in a sample can be tagged with a sufficient number of different tags so that there is a high probability (e.g., at least 90%, at least 95%, at least 98%, at least 99%, at least 99.9% or at least 99.99%) that all polynucleotides mapping to a particular genomic region bear a different identifying tag (molecules within the region are substantially uniquely tagged). The genomic region to which the polynucleotides map can be, for example, (1) the entire panel of genes being sequenced, (2) some portion of that panel, such as mapping within a single gene, exon or intron, (3) a single nucleotide coordinate (e.g., at least one nucleotide in the polynucleotide maps to the coordinate, for example, the start position, stop position, mid-point or anywhere between) or (4) a particular pair of start/stop (begin/end) nucleotide coordinates. The number of different identifiers (tag counts) necessary to substantially uniquely tag polynucleotides is a function of how many original polynucleotide molecules in the sample that map to the region. This, in turn, is a function of several factors. One factor is the total number of haploid genome equivalents included in the assay. Another factor is the average size of the polynucleotide molecules. Another factor is the distribution of the molecules across the region. This, in turn, can be a function of the cleavage pattern - one may expect cleavage to occur primarily between nucleosomes so that more polynucleotides map across a nucleosome location than between nucleosomes. Another factor is the distribution of barcodes in the pool and the ligation efficiency of individual barcodes, potentially causing differences in affective concentration of one barcode versus another. Another factor is the size of the region within which the molecules to be uniquely tagged are confined (e.g., same start/stop or same exon).

The identifier can be a single barcode attached to one end of a molecule, or two barcodes, each attached to different ends of the molecule. Attaching barcodes independently to both ends of a molecule increases by square the number of possible identifiers. In this case the number of different barcodes is selected such that the combination of barcodes on each end of a particular polynucleotide has a high probability of being unique with respect to other polynucleotides mapping to the same selected genomic region.

In certain embodiments, the number of different identifiers or barcode combinations (tag count) used can be at least any of 64, 100, 400, 900, 1400, 2500, 5625, 10,000, 14,400, 22,500 or 40,000 and no more than any of 90,000, 40,000, 22,500, 14,400 or 10,000. For example, the number of identifiers or barcode combinations can be between 64 and 90,000, between 400 and 22,500, 400 and 14,400 or between 900 and 14,400.

In a sample comprising fragmented genomic DNA, e.g., cell-free DNA (cfDNA), from a plurality of genomes, there is some likelihood that more than one polynucleotide from different genomes will have the same start and stop positions ("duplicates" or "cognates"). The probable number of duplicates beginning at any position is a function of the number of haploid genome equivalents in a sample and the distribution of fragment sizes. For example, cfDNA has a peak of fragments at about 160 nucleotides, and most of the fragments in this peak range from about 140 nucleotides to 180 nucleotides. Accordingly, cfDNA from a genome of about 3 billion bases (e.g., the human genome) may be comprised of almost 20 million (2×10⁷) polynucleotide fragments. A sample of about 30 ng DNA can contain about 10,000 haploid human genome equivalents. (Similarly, a sample of about 100 ng of DNA can contain about 30,000 haploid human genome equivalents.) A sample containing about 10,000 (10⁴) haploid genome equivalents of such DNA can have about 200 billion (2×10¹¹) individual polynucleotide molecules. It has been empirically determined that in a sample of about 10,000 haploid genome equivalents of human DNA, there are about 3 duplicate polynucleotides beginning at any given position. Thus, such a collection can contain a diversity of about 6×10¹⁰-8×10¹⁰ (about 60 billion-80 billion e.g., about 70 billion (7×10¹⁰)) differently sequenced polynucleotide molecules.

The probability of correctly identifying molecules is dependent on initial number of genome equivalents, the length distribution of sequenced molecules, sequence uniformity and number of tags. The number can be calculated using a Poisson distribution. When the tag count is equal to one, that is, equivalent to having no unique tags or not tagging. Table 1 below lists the probability of correctly identifying a molecule as unique assuming a typical cell-free size distribution as above.

**Table 1**

| Tag Count | Tag %Correctly uniquely identified |
|---|---|
| 1000 human haploid genome equivalents | |
| 1 | 96.9643 |
| 4 | 99.2290 |
| 9 | 99.6539 |
| 16 | 99.8064 |
| 25 | 99.8741 |
| 100 | 99.9685 |
| | |
| 3000 human haploid genome equivalents | |
| 1 | 91.7233 |
| 4 | 97.8178 |
| 9 | 99.0198 |
| 16 | 99.4424 |
| 25 | 99.6412 |
| 100 | 99.9107 |

In this case, upon sequencing the genomic DNA, it may not be possible to determine which sequence reads are derived from which parent molecules. This problem can be diminished by tagging parent molecules with a sufficient number of unique identifiers (e.g., the tag count) such that there is a likelihood that two duplicate molecules, i.e., molecules having the same start and stop positions, bear different unique identifiers so that sequence reads are traceable back to particular parent molecules. One approach to this problem is to uniquely tag every, or nearly every, different parent molecule in the sample. However, depending on the number of haploid gene equivalents and distribution of fragment sizes in the sample, this may require billions of different unique identifiers.

This method can be cumbersome and expensive. A population of polynucleotides in a sample of fragmented genomic DNA may be tagged with n different unique identifiers, wherein n is at least 2 and no more than 100,000*z, wherein z is a measure of central tendency (e.g., mean, median, mode) of an expected number of duplicate molecules having the same start and stop positions. n may be at least any of 2^{∗}z, 3*z, 4*z, 5*z, 6*z, 7*z, 8*z, 9*z, 10*z, 11*z, 12*z, 13*z, 14*z, 15*z, 16*z, 17*z, 18*z, 19*z, 20*z or 100*z (e.g., lower limit). n may be no greater than 100,000*z, 10,000*z, 2000*z, 1000*z, 500*z or 100*z (e.g., upper limit). Thus, n can range between any combination of these lower and upper limits. n may be between 100*z and 1000*z, 5*z and 15*z, between 8*z and 12*z, or about 10*z. For example, a haploid human genome equivalent has about 3 picograms of DNA. A sample of about 1 microgram of DNA contains about 300,000 haploid human genome equivalents. The number n can be between 15 and 45, between 24 and 36, between 64 and 2500, between 625 and 31,000, or about 900 and 4000. Improvements in sequencing can be achieved as long as at least some of the duplicate or cognate polynucleotides bear unique identifiers, that is, bear different tags. However, the number of tags used may be selected so that there is at least a 95% chance that all duplicate molecules starting at any one position bear unique identifiers. For example, a sample comprising about 10,000 haploid human genome equivalents of cfDNA can be tagged with about 36 unique identifiers. The unique identifiers can comprise six unique DNA barcodes. Attached to both ends of a polynucleotide, 36 possible unique identifiers are produced. Samples tagged in such a way can be those with a range of about 10 ng to any of about 100 ng, about 1 µg, about 10 µg of fragmented polynucleotides, e.g., genomic DNA, e.g. cfDNA.

Accordingly, compositions of tagged polynucleotides can be obtained using kits of the invention. The polynucleotides can comprise fragmented DNA, e.g., cfDNA. A set of polynucleotides in the composition that map to a mappable base position in a genome can be non-uniquely tagged, that is, the number of different identifiers can be at least at least 2 and fewer than the number of polynucleotides that map to the mappable base position. A composition of between about 10 ng to about 10 µg (e.g., any of about 10 ng-1 µg, about 10 ng-100 ng, about 100 ng-10 µg, about 100 ng-1 µg, about 1 µg-10 µg) can bear between any of 2, 5, 10, 50 or 100 to any of 100, 1000, 10,000 or 100,000 different identifiers. For example, between 5 and 100 or between 100 and 4000 different identifiers can be used to tag the polynucleotides in such a composition.

Events in which different molecules mapping to the same coordinate (in this case having the same start/stop positions) and bearing the same, rather than different, tags, are referred to as "molecular collisions". In certain instances, the actual number of molecular collisions may be greater than the number of theoretical collisions, calculated, e.g., as above. This may be a function of uneven distribution of molecules across coordinates, differences in efficiency of ligation between barcodes, and other factors. In this case, empirical methods can be used to determine the number of barcodes needed to approach the theoretical collision number. For example, a method of determining a number of barcodes required to diminish barcode collisions for a given haploid genome equivalent may be based on length distribution of sequenced molecules and sequence uniformity. The method comprising creating a plurality of pools of nucleic acid molecules; tagging each pool with incrementally increasing numbers of barcodes; and determining an optimal number of barcodes that reduces the number of barcode collisions to a theoretical level, e.g., that could be due to differences in affective barcode concentrations due to differences is pooling and ligation efficiency.

The number of identifiers necessary to substantially uniquely tag polynucleotides mapping to a region can be determined empirically. For example, a selected number of different identifiers can be attached to molecules in a sample, and the number of different identifiers for molecules mapping to the region can be counted. If an insufficient number of identifiers is used, some polynucleotides mapping to the region will bear the same identifier. In that case, the number identifiers counted will be less than the number of original molecules in the sample. The number of different identifiers used can be iteratively increased for a sample type until no additional identifiers, representing new original molecules, are detected. For example, in a first iteration, five different identifiers may be counted, representing at least five different original molecules. In a second iteration, using more barcodes, seven different identifiers are counted, representing at least seven different original molecules. In a third iteration, using more barcodes, 10 different identifiers are counted, representing at least ten different original molecules. In a fourth iteration, using more barcodes, 10 different identifiers, again, are counted. At this point, adding more barcodes is not likely to increase the number of original molecules detected.

### 6. Sequencing

Sample nucleic acids flanked by adapters with or without prior amplification can be subject to sequencing. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next generation sequencing, Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may multiple lanes, multiple channels, multiple wells, or other mean of processing multiple sample sets substantially simultaneously. Sample processing unit can also include multiple sample chambers to enable processing of multiple runs simultaneously.

The sequencing reactions can be performed on one more fragments types known to contain markers of cancer of other disease. The sequencing reactions can also be performed on any nucleic acid fragments present in the sample. The sequence reactions may provide for sequence coverage of the genome of at least 5%, 10%, 15%, 20%, 25%, 30%, 40%, 50%, 60%, 70%, 80%, 90%, 95%, 99%, 99.9% or 100%. In other cases, sequence coverage of the genome may be less than 5%, 10%, 15%, 20%, 25%, 30%, 40%, 50%, 60%, 70%, 80%, 90%, 95%, 99%, 99.9% or 100%.

Simultaneous sequencing reactions may be performed using multiplex sequencing. In some cases, cell-free nucleic acids may be sequenced with at least 1000, 2000, 3000, 4000, 5000, 6000, 7000, 8000, 9000, 10000, 50000, 100,000 sequencing reactions. In other cases, cell-free polynucleotides may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions.

The sequencing method can be massively parallel sequencing, that is, simultaneously (or in rapid succession) sequencing any of at least 100, 1000, 10,000, 100,000, 1 million, 10 million, 100 million, or 1 billion nucleic acid molecules.

### 7. Analysis

Kits of the invention may be useful in methods used to diagnose presence of conditions, particularly cancer, in a subject, to characterize conditions (e.g., staging cancer or determining heterogeneity of a cancer), monitor response to treatment of a condition, effect prognosis risk of developing a condition or subsequent course of a condition.

Various cancers may be detected using these methods. Cancers cells, as most cells, can be characterized by a rate of turnover, in which old cells die and replaced by newer cells. Generally dead cells, in contact with vasculature in a given subject, may release DNA or fragments of DNA into the blood stream. This is also true of cancer cells during various stages of the disease. Cancer cells may also be characterized, dependent on the stage of the disease, by various genetic aberrations such as copy number variation as well as rare mutations. This phenomenon may be used to detect the presence or absence of cancers individuals using these methods.

The types and number of cancers that may be detected may include blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like.

Cancers can be detected from genetic variations including mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, abnormal changes in nucleic acid methylation infection and cancer.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers progress, becoming more aggressive and genetically unstable. Other cancers may remain benign, inactive or dormant. Kits of the invention may be useful in methods that may be useful in determining disease progression.

The present analysis is also useful in determining the efficacy of a particular treatment option. Successful treatment options may increase the amount of copy number variation or rare mutations detected in subject's blood if the treatment is successful as more cancers may die and shed DNA. In other examples, this may not occur. In another example, perhaps certain treatment options may be correlated with genetic profiles of cancers over time. This correlation may be useful in selecting a therapy. Additionally, if a cancer is observed to be in remission after treatment, kits of the invention may be useful in methods used to monitor residual disease or recurrence of disease.

Kits of the invention may be useful in methods used for detecting genetic variations in conditions other than cancer. Immune cells, such as B cells, may undergo rapid clonal expansion upon the presence certain diseases. Clonal expansions may be monitored using copy number variation detection and certain immune states may be monitored. In this example, copy number variation analysis may be performed over time to produce a profile of how a particular disease may be progressing. Copy number variation or even rare mutation detection may be used to determine how a population of pathogens are changing during the course of infection. This may be particularly important during chronic infections, such as HIV/AIDs or Hepatitis infections, whereby viruses may change life cycle state and/or mutate into more virulent forms during the course of infection. Kits of the invention may be useful in methods used to determine or profile rejection activities of the host body, as immune cells attempt to destroy transplanted tissue to monitor the status of transplanted tissue as well as altering the course of treatment or prevention of rejection.

Further, kits of the invention may be useful in methods used to characterize the heterogeneity of an abnormal condition in a subject, the method comprising generating a genetic profile of extracellular polynucleotides in the subject, wherein the genetic profile comprises a plurality of data resulting from copy number variation and rare mutation analyses. In some cases, including but not limited to cancer, a disease may be heterogeneous. Disease cells may not be identical. In the example of cancer, some tumors are known to comprise different types of tumor cells, some cells in different stages of the cancer. In other examples, heterogeneity may comprise multiple foci of disease. Again, in the example of cancer, there may be multiple tumor foci, perhaps where one or more foci are the result of metastases that have spread from a primary site.

Kits of the invention may be useful in methods used to generate or profile, fingerprint or set of data that is a summation of genetic information derived from different cells in a heterogeneous disease. This set of data may comprise copy number variation and rare mutation analyses alone or in combination.

Kits of the invention may be useful in methods used to diagnose, prognose, monitor or observe cancers or other diseases of fetal origin. That is, these methodologies may be employed in a pregnant subject to diagnose, prognose, monitor or observe cancers or other diseases in a unborn subject whose DNA and other nucleic acids may co-circulate with maternal molecules.

### 9. Kits

Kits of the invention can be for practice of any of the above methods. Kits of the invention include a pair of at least partially double-stranded adapters with T and C single nucleotide 3' tails respectively. Preferably the paired oligonucleotides are identical except for the T and C tails. Optionally, the kit is free of at least partially double-stranded adapters with A and G single nucleotide 3' tails. Preferably the adapters are Y shaped such as adapters comprising oligonucleotides of SEQ ID NOS. 1 and 2, and 3 and 2. Kits can also include enzymes for practice of the methods, such as T4 polymerase or Klenow large fragment, and/or Taq polymerase, and optionally the four standard nucleotide types. Kits can also include packaging, leaflets, CDs or the like providing instructions for practice of the claimed methods.

### Examples

The use of C- and T- tailed adapters contributed to increased sensitivity by capturing more molecules in a sample. C-adapters were tested in ratios varying from 0 to 1:2.75 (36%) relative to T adapters as shown in Table 2 below.

**Table 2**

| Sample # | Input (ng) | T-tailed (40uM) | C-tailed (40 uM) | %LIG |
|---|---|---|---|---|
| 1 | 20 | 3.25 | 0.5 | 80% |
| 2 | 20 | 3.25 | 0.5 | 77% |
| 3 | 20 | 3.25 | 1 | 79% |
| 4 | 20 | 3.25 | 1 | 80% |
| 5 | 20 | 2.75 | 0.5 | 79% |
| 6 | 20 | 2.75 | 0.5 | 77% |
| 7 | 20 | 2.75 | 1 | 80% |
| 8 | 20 | 2.75 | 1 | 78% |
| 9 | 20 | 3.25 | - | 75% |
| 10 | 20 | 3.25 | - | 75% |

All samples in which C-tailed adapters were present showed a higher yield of nucleic acids ligated to adapters (% ligation) than samples in which C-tails were absent. The best yield was for C-tailed to T-tailed primers in a ratio of 1:3.25 (about 30%) but improved yields were obtained in ratios from 0.5:3.25 (about 15%) to 1:2.75 (36%)

After sequencing of amplified DNA, diversity was calculated for each preparation. Diversity is the number of molecules sequenced, calculated by: (avg DNA molecule size in bp) * (# of unique molecules sequenced) / (targeted region size in bp). The diversity was generally greater in the samples in which C-tailed adaptor was present. Sequencing also indicated the proportion of T to C-tailed adaptors incorporated was about 10%.

## Claims

1. A kit comprising:
at least partially double-stranded adapters with a single nucleotide T overhang at a 3' end (T-tailed adapters); and
at least partially double-stranded adapters with a single nucleotide C overhang at a 3' end (C-tailed adapters).

2. The kit of claim 1, wherein the molar ratio of at least partially double-stranded adapters with a single nucleotide T overhang to a single nucleotide C overhang is 4:1 to 2:1, for example 3:1.

3. The kit of claim 1, wherein the percentage of C-tailed adapters relative to T-tailed adapters ranges from about 5-40% by moles, for example, 10-35%, 15-25%, 20-35%, 25-35% or about 30%.

4. The kit of any one of the preceding claims, further comprising:
at least partially double-stranded blunt-ended adapters.

5. The kit of claim 4, wherein the molar ratio of blunt-ended adapters to tailed adapters is 1:5 to 1:500, for example 1:10 to 1:100.

6. The kit of claim 4, wherein blunt-ended adapters are at a molar ratio of 0.2-20%, or 0.5-15%, or 1-10% of adapters with T- and C-tailed adapters.

7. The kit of any one of the preceding claims, further comprising
a T4 polymerase or Klenow large fragment; and/or
a Taq polymerase.

8. The kit of claim 7, further comprising four standard nucleotide types.

9. The kit of any one of the preceding claims, wherein the kit is free of at least partially double-stranded adapters with A single nucleotide 3' tails, and at least partially double-stranded adapters with G single nucleotide 3' tails.

10. The kit of any one of the preceding claims, wherein the adapters are Y-shaped adapters.

11. The kit of claim 10, wherein the adapters comprise oligonucleotides of SEQ ID NOs. 1 and 2, and 3 and 2.

12. The kit of any one of the preceding claims, wherein the adapters comprise a primer binding site and/or a barcode.

13. The kit of claim 12, wherein the barcodes provide between 64 and 90,000, between 400 and 22,500, between 400 and 14,400, or between 900 and 14,400 different barcode combinations.

## Patentansprüche

1. Kit, umfassend:
wenigstens teilweise doppelsträngige Adapter mit einem Einzelnukleotid-T-Überhang an einem 3'-Ende (Adapter mit T-Tail); und
wenigstens teilweise doppelsträngige Adapter mit einem Einzelnukleotid-C-Überhang an einem 3'-Ende (Adapter mit C-Tail).

2. Kit nach Anspruch 1, wobei das Molverhältnis von wenigstens teilweise doppelsträngigen Adaptern mit einem Einzelnukleotid-T-Überhang zu einem Einzelnukleotid-C-Überhang 4:1 bis 2:1, beispielsweise 3:1, beträgt.

3. Kit nach Anspruch 1, wobei der Prozentsatz Adapter mit C-Tail relativ zu Adaptern mit T-Tail im Bereich von 5-40 Mol-%, beispielsweise 10-35%, 15-25%, 20-35%, 25-35% oder etwa 30%, liegt.

4. Kit nach einem der vorhergehenden Ansprüche, ferner umfassend:
wenigstens teilweise doppelsträngige Adapter mit stumpfem Ende.

5. Kit nach Anspruch 4, wobei das Molverhältnis von Adaptern mit stumpfem Ende zu Adaptern mit Tail 1:5 bis 1:500, beispielsweise 1:10 bis 1:100, beträgt.

6. Kit nach Anspruch 4, wobei Adapter mit stumpfem Ende in einem Molverhältnis von 0,2-20% oder 0,5-15% oder 1-10% Adapter mit Adaptern mit T- und C-Tail vorliegen.

7. Kit nach einem der vorhergehenden Ansprüche, ferner umfassend:
eine T4-Polymerase bzw. ein großes Klenow-Fragment; und/oder
eine Taq-Polymerase.

8. Kit nach Anspruch 7, ferner umfassend vier Nukleotid-Standardtypen.

9. Kit nach einem der vorhergehenden Ansprüche, wobei das Kit frei von wenigstens teilweise doppelsträngigen Adaptern mit A-Einzelnukleotid-3'-Tails und wenigstens teilweise doppelsträngigen Adaptern mit G-Einzelnukleotid-3'-Tails ist.

10. Kit nach einem der vorhergehenden Ansprüche, wobei es sich bei den Adaptern um Y-förmige Adapter handelt.

11. Kit nach Anspruch 10, wobei die Adapter Oligonukleotide unter SEQ ID NO. 1 und 2 sowie 3 und 2 umfassen.

12. Kit nach einem der vorhergehenden Ansprüche, wobei die Adapter eine Primer-Bindungsstelle und/oder einen Barcode umfassen.

13. Kit nach Anspruch 12, wobei mit den Barcodes zwischen 64 und 90 000, zwischen 400 und 22 500, zwischen 400 und 14 400 oder zwischen 900 und 14 400 unterschiedliche Barcodekombinationen bereitgestellt werden.

## Revendications

1. Trousse comprenant :
des adaptateurs, en double brin au moins partiellement, avec un surplomb de T d'un seul nucléotide à une extrémité 3' (adaptateurs à queue de T) ; et
des adaptateurs, en double brin au moins partiellement, avec un surplomb de C d'un seul nucléotide à une extrémité 3' (adaptateurs à queue de C) .

2. Trousse de la revendication 1, dans laquelle le rapport molaire des adaptateurs en double brin au moins partiellement avec un surplomb de T d'un seul nucléotide par rapport à un surplomb de C d'un seul nucléotide est de 4:1 à 2:1, par exemple 3:1.

3. Trousse de la revendication 1, dans laquelle le pourcentage d'adaptateurs à queue de C par rapport à des adaptateurs à queue de T est compris dans une gamme de 5-40% environ en moles, par exemple 10-35%, 15-25%, 20-35%, 25-35% ou environ 30%.

4. Trousse de l'une quelconque des revendications précédentes, comprenant en outre :
des adaptateurs, en double brin au moins partiellement, à extrémités franches.

5. Trousse de la revendication 4, dans laquelle le rapport molaire des adaptateurs à extrémités franches par rapport aux adaptateurs ayant une queue est de 1:5 à 1:500, par exemple de 1:10 à 1:100.

6. Trousse de la revendication 4, dans laquelle les adaptateurs à extrémités franches se trouvent à un rapport molaire de 0,2-20% ou de 0,5-15% ou de 1-10% des adaptateurs avec des adaptateurs à queues de T et de C.

7. Trousse de l'une quelconque des revendications précédentes, comprenant en outre
une polymérase T4 ou un grand fragment de Klenow ; et/ou
une polymérase Taq.

8. Trousse de la revendication 7, comprenant en outre quatre types de nucléotides standards.

9. Trousse de l'une quelconque des revendications précédentes, la trousse étant dépourvue d'adaptateurs, en double brin au moins partiellement, avec des queues en 3' à un seul nucléotide A et d'adaptateurs, en double brin au moins partiellement, avec des queues en 3' à un seul nucléotide G.

10. Trousse de l'une quelconque des revendications précédentes, dans laquelle les adaptateurs sont des adaptateurs en forme de Y.

11. Trousse de la revendication 10, dans laquelle les adaptateurs comprennent des oligonucléotides de SEQ ID n° : 1 et 2 ainsi que 3 et 2.

12. Trousse de l'une quelconque des revendications précédentes, dans laquelle les adaptateurs comprennent un site de liaison à une amorce et/ou un code-barres.

13. Trousse de la revendication 12, dans laquelle les codes-barres fournissent entre 64 et 90 000, entre 400 et 22 500, entre 400 et 14 400 ou entre 900 et 14 400 combinaisons de codes-barres différents.
